# EUROPEAN PATENT SPECIFICATION

(11) **EP 1 399 143 B1**
(45) Date of publication and mention of the grant of the patent: **15.11.2006**
(21) Application number: 02741156.0
(22) Date of filing: 24.05.2002
(51) Int. Cl.: A61K 31/221, A61P 25/18, A61P 25/24

(54) **USE OF ACETYL L-CARNITINE FOR THE PREPARATION OF A MEDICATION TO TREAT ANHEDONIA**
VERWENDUNG VON L-ACETYLCARNITIN ZUR HERSTELLUNG EINES MEDIKAMENTS ZUR BEHANDLUNG VON ANHEDONIA
UTILISATION DE L'ACETYL L-CARNITINE POUR LA PREPARATION D'UNE MEDICATION DESTINEE AU TRAITEMENT DE L'ANHEDONIE

(30) Priority: 29.05.2001 IT RM20010029; 08.06.2001 IT RM20010031
(43) Date of publication of application: 24.03.2004
(73) Proprietor: SIGMA-TAU Industrie Farmaceutiche Riunite S.p.A., 00144 Roma (IT)
(72) Inventor: CALVANI, Menotti, Sigma-Tau Ind. Farm. Riunite SpA, I-00040 Pomezia (IT); MOSCONI, Luigi, Sigma-Tau Ind. Farm. Riunite SpA, I-00040 Pomezia (IT)
(74) Representative: Spadaro, Marco
(86) International application number: PCT/IT2002/000339
(87) International publication number: WO 2002/096411

(56) References cited:
- WO-A-98/57629
- BRUNELLO N ET AL: "Dysthymia: Clinical picture, extent of overlap with chronic fatigue syndrome, neuropharmacological considerations, and new therapeutic vistas." JOURNAL OF AFFECTIVE DISORDERS, vol. 52, no. 1-3, January 1999 (1999-01), pages 275-290, XP002214518 ISSN: 0165-0327
- SCUCCIMARRA A ET AL: "L ACETYLCARNITINE IN DEPRESSIVE SYNDROMES OF ALL AGES" GAZZETTA MEDICA ITALIANA ARCHIVIO PER LE SCIENZE MEDICHE, vol. 147, no. 6, 1988, pages 213-214, XP001105518 ISSN: 0393-3660
- AURIACOMBE M ET AL: "Animal models of anhedonia." PSYCHOPHARMACOLOGY. GERMANY DEC 1997, vol. 134, no. 4, December 1997 (1997-12), pages 337-338, XP002214519 ISSN: 0033-3158
- TEMPESTA E ET AL: "L-ACETYLCARNITINE IN DEPRESSED ELDERLY SUBJECTS. A CROSS-OVER STUDY VS PLACEBO" DRUGS UNDER EXPERIMENTAL AND CLINICAL RESEARCH, BIOSCIENCE EDIPRINT INC, XX, vol. 13, no. 7, 1987, pages 417-423, XP000874764 ISSN: 0378-6501

## Description

The present invention relates to the use of acetyl L-carnitine for the preparation of a medicament for the treatment of anhedonia.

Anhedonia is an aspect of personality usually present in patients with schizophrenia and other pathologies (Can. Psychiatr. Assoc. J. 1978 Nov; 23 (7):487-92) characterized by the reduced sensitivity to stimuli that patients once enjoyed.

J. Clin. Pharm. Ther. 2000 Oct; 25(S):363-71 discloses the use of the sertraline for the treatment of such pathology.

Psychopharmacology (Berl) 1992; 109(4):433-8 discloses the use of the fluoxetine and the maprolitine for the treatment of anhedonia.

Though commonly used, both the psychotherapy and the psychoactive medicaments are not deemed useful for the treatment of such pathology (Can. Psychiatr. Assoc. J. 1978 Nov; 23 (7):487-92). Further medicaments that can be useful for the treatment of anhedonia are not known.

Owing to the fact that only few medicaments are available for the treatment of anhedonia, and considering that both psychology and psychoactive medications are ineffective, in the medical field there is a great need for medicaments useful for the treatment of such pathology.

Now it has been found that acetyl L-carnitine is useful agents for the treatment of anhedonia.

Therefore, an object of the present invention is the use of acetyl L-carnitine, or a pharmaceutically acceptable salt thereof, for the preparation of a medicament for the treatment of anhedonia.

By pharmaceutically acceptable salt of acetyl L-carnitine it is meant any of its salt with an acid that does not give rise to undesirable toxic or side effects. These acids are well known to pharmacologists and to experts in pharmacy.

Example of these salts are for example: chloride, bromide, orotate, acid aspartate, acid citrate, citrate magnesium, acid phosphate, fumarate and acid fumarate, fumarate magnesium, lactate, maleate and acid maleate, mucate, acid oxalate, pamoate, acid pamoate, acid sulphate, phosphate glucose, tartrate, acid tartrate, tartrate magnesium, 2-amine ethanesulphonate, magnesium 2-amine ethanesulphonate, tartrate coline and trichloroacetate.

The following examples better illustrate the present invention.In the course of the experimental tests herein below, the effects of the acetyl L-carnitine have been studied according to an experimental paradigm based on anhedonia, a model of a particular symptom of chronic stress.

The anhedonia model is based on the observation that the fact of being exposed to a repeated unavoidable stress prevents the development of an appetitive behaviour induced and maintained by a highly palatable food (vanilla sugar) in rats fed ad libitum (Behav. Pharmacol. 8:619-628, 1997).

The animals treated with acetyl L-carnitine showed to be completely protected against the negative effects of stress on the vanilla sugar induced/maintained acquisition of an appetitive behaviour (VAB) by preventing the dopaminergic transmission from decreasing in the nucleus accumbens (Nacs) as a result of stress.

Other known antidepressant medicaments such as the imipramine (IMI) and the fluoxetine (FLX) (Am. J. Psychiatry. 2000 Mar; 157(3):344-50) were tested together with the acetyl L-carnitine in the course of these experiments.

Unexpectedly, it has been found that acetyl L-carnitine is the only effective medicament to antagonize the decreasing effect of repeated stress exposure on dopaminergic transmission in the Nacs. Thus, the acetyl L-carnitine enables the animals to perceive palatable food as a sufficient reinforcement to sustain motivated appetitive behaviour.

### EXAMPLE 1

### Long-term effect of acetyl L-carnitine on the acquisition of appetitive behaviour.

Two experiments were carried out. The first experiment aimed at investigating whether the treatment with acetyl L-carnitine may prevent the negative effect of chronic stress on the acquisition of appetitive behaviour sustained by vanilla sugar (VAB) in rats.

In this experiment, the rats were treated with physiological solution (PS) or acetyl L-carnitine (ALCAR). The treatments started 2 weeks before the training trial and continued throughout the period of the experiment, as described herein below.

Forty Sprague Dawley rats weighing approximately 250 grams were divided into 4 groups according to the following protocol:
a) VAB, [control group (CTR)]: 10 rats were treated with physiological solution (1 ml/kg IP) twice a day (BID) for 14 days. Then, a three-week training trial in a Y-maze was started (Y maze) (Brain. Res. Protocols 7(1):11-20; 2001).
b) acetyl L-carnitine: 10 rats were treated with acetyl L-carnitine (10 mg/kg IP, BID, in a volume of 1 ml/kg) for 14 days. Then the training trial in the Y-maze was started;
c) Stress: 10 rats were treated with physiological solution (1 ml/kg IP, BID) for 14 days. Then, the animals were exposed to the sequence of pre-test [with the pre-test, the animals were exposed to both unavoidable stress (electric shocks) of minimum intensity and duration, capable to induce a reliable modification of behaviour. Brain. Res. Protocols 7(1):11-20; 2001]; escape test [(also defined as "test") carried out 24 hours after the pre-test, to assess the induced behaviour modification (Brain. Res. Protocols 7(1):11-20; 2001)]; Y-maze training and exposure to the chronic stress procedure on alternate days (maze one day, and stress the next day) for three weeks (Brain. Res. Protocols 7(1):11-20; 2001), while continuing treatment with physiological solution;
d) acetyl L-carnitine + Stress: 10 rats were treated with acetyl L-carnitine (10 mg/kg, IP, BID) for 14 days. Then, animals were exposed to the sequence of: - pre-test; - test; - Y-maze training and simultaneously exposed to the chronic stress procedure on alternate days (maze one day, and stress the next day) for three weeks, while continuing treatment with acetyl L-carnitine.
   The Y-maze was made up of three wings. The vanilla sugar for the acquisition of the appetitive behaviour was in one of the two diverging wings (VAB training, 3 weeks).
   At the end of the VAB training, all the animals exposed to stress underwent the escape test (Brain. Res. Protocols 7(1):11-20; 2001).
   The second experiment aimed at determining whether the treatment with L-carnitine would antagonize the negative effect of chronic stress on the VAB acquisition in rats.
   Forty rats were divided in 4 groups, each one consisting of 10 animals, as follows:
e) VAB: the physiological solution was administered to the animals (1 ml/kg IP, BID) for 8 days, and then the rats were trained in the Y-maze for VAB acquisition while continuing the treatment with physiological solution for 3 weeks;
f) Stress + VAB: the animals were exposed to the pre-test. Twenty-four hours later they were subjected to the escape test, and then exposed to the chronic stress procedure for 7 days. On day 9, they started their training in the Y-maze while exposed to stress on alternate days;
g) acetyl L-carnitine + Stress + VAB: the animals were exposed to the pre-test. Twenty-four hours later they underwent the escape test. Then, they were exposed to the protocol of chronic stress for 7 days, while receiving acetyl L-carnitine 10 mg/kg IP, BID for 8 days. On day 9, the rats started their VAB training in the Y-maze and exposure to stress on alternate days, while continuing treatment with the acetyl L-carnitine for 3 weeks;
h) IMI + Stress + VAB: the animals were exposed to the pre-test. Twenty-four hours later, they underwent the escape test, and then they were exposed to the chronic stress protocol for 7 days while receiving imipramine 5 mg/kg IP, BID for 8 days. On day 9, the rats started their training in the Y-maze and exposure to stress on alternate days, while continuing with IMI for 3 weeks.

At the end of the training period, all the animals underwent the escape test.

Two days later, animals were implanted with microdialysis probes into the nucleus accumbens (Nacs) (Brain. Res. Protocols 5(1):16-24: 2000) and underwent dialysis on the next day.

Once the dopamine basal level had been reached, the rats were administered a single dose of cocaine 5 mg/kg IP in 0.1 ml volume of water, and dialysed within the next 60 minutes.

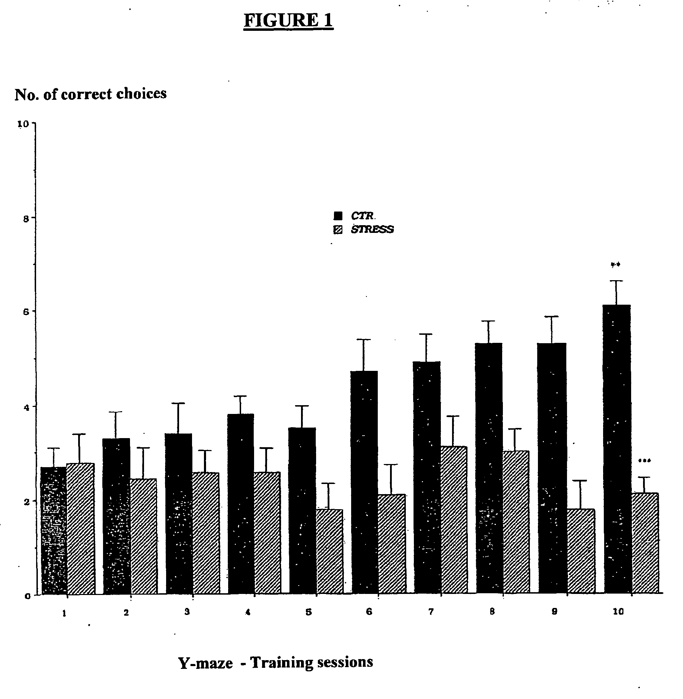

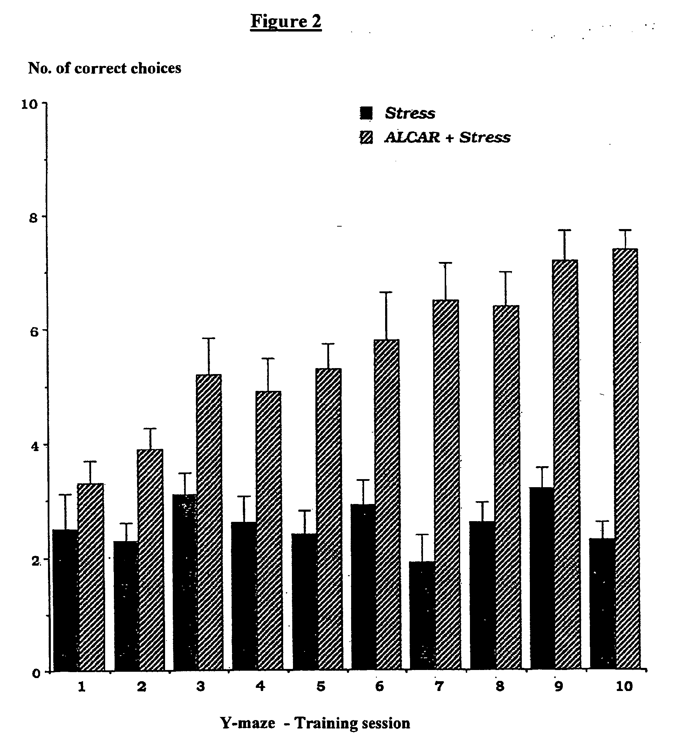

The results, reported in Figures 1 and 2, show the VAB acquisition of control rats and rats treated with acetyl L-carnitine in comparison to rats exposed to chronic stress.

It is clear that the exposure to chronic stress has a negative effect on the acquisition of motivated appetitive behaviour.

Furthermore, the 14-day pre-treatment with acetyl L-carnitine, followed by the administration of acetyl L-carnitine throughout the training phase in association with the exposure to chronic stress have prevented such negative effect from occurring. Finally, the rats acquiring VAB under the effect of the acetyl L-carnitine recovered in full their capacity to avoid negative stimuli. The results are shown in Table 1.

**TABLE 1**

| **Group** | **No. of escapes** | **No. of escapes** |
|---|---|---|
| | 24 hours after the pre-test | After training for VAB and exposure to stress |
| Naive# | 26.3±0.4 | |
| Stress | 1.4±0.7*** | 2.7±0.9*** |
| ALCAR + Stress^{§} | 25.8±2.5 | 26.4±2.9 |

| | | |
|---|---|---|
| # = The group defined as Naive was subjected to the escape test, but was not subjected to the chronic stress procedure. § = treated with acetyl L-carnitine 10 mg/kg IP, BID for 14 days The values represent the mean ± standard error. *** = p<0.001 compared with the Naive group and the acetyl L-carnitine + Stress group. | | |

The results obtained show that the acetyl L-carnitine has an anti-anhedonic effect superimposable to the known effects of IMI and FLX (Brain. Res. Protocols 7(1):11-20; 2001).

Nevertheless, the correct conclusion deriving from a parallel comparison between the experiments with acetyl L-carnitine herein described and the known experiments with IMI and FLX is that the administration of such substances to the rats for a period of 1-3 weeks before exposing them to the chronic stress procedure prevents the behaviour sequelae induced by stress from occurring. However, the fact that such compounds prevented the development of the chronic stress negative effects on VAB acquisition did not necessarily mean that they would have antagonized the effect of stress on motivated appetitive behaviour. The second experimental protocol described above was carried out to investigate this point.

The results reported in the following Table 2 show that:
1) the rats exposed to chronic stress (Stress) do not acquire VAB;
2) chronic administration of IMI during exposure to stress and training in the Y-maze protected the rats from the negative effect of chronic stress on VAB acquisition.
3) chronic administration of acetyl L-carnitine during exposure to stress and training in the Y-maze have fully protected the rats from negative effect of chronic stress on VAB acquisition.

**TABLE 2**

| **Group** | **No. of correct choices** |
|---|---|
| VAB | 6.6±0.3 |
| Stress + VAB | 2.4±0.8*** |
| IMI + Stress + VAB | 2.9±1.2*** |
| ALCAR + Stress + VAB | 7.6±0.5 |

| | |
|---|---|
| ALCAR 10 mg/kg IP, BID or IMI 5 mg/kg BID, plus exposure to chronic stress for 8 days; on day 9, training in the Y-maze for VAB, 3-week treatment with ALCAR or IMI and exposure to stress, on alternate days. The values represent the mean ± standard error. *** = p<0.001 compared with VAB group and ALCAR + Stress + VAB group. | |

The results of table 3 show that the rats belonging to the following groups: Stress + VAB, IMI + Stress and acetyl L-carnitine + Stress have an acute escape deficit the day before the VAB training procedure startup. Furthermore, the results show that both IMI and acetyl L-carnitine completely reversed the escaped deficit induced by chronic stress.

**Table 3**

| **Group** | **N° of escapes** | **N° of escapes** |
|---|---|---|
| | After 8 days | After stress + 3-week VAB training |
| Naive# | 24.7±0.5 | |
| Stress + VAB | 1.6±0.6*** | |
| ALCAR + Stress + VAB | 1.0±0.5*** | 22.4±2.8 |
| IMI + Stress + VAB | 2.5±1.1*** | 24.8±1.0 |

| | | |
|---|---|---|
| # = The Naive group was subjected to the escape test, but not to the chronic stress procedure. ALCAR 10 mg/kg IP, BID or IMI 5 mg/kg BID, plus exposure to chronic stress for 8 days; from day 9, training in the Y-maze for VAB (3 weeks), while both the three-week treatment with ALCAR or IMI and the exposure to stress on alternate days continued. The values represent the mean ± standard error. *** = p<0.001 compared with the Naive group, ALCAR + Stress + VAB group, and IMI + Stress + VAB group at the end of the training period. | | |

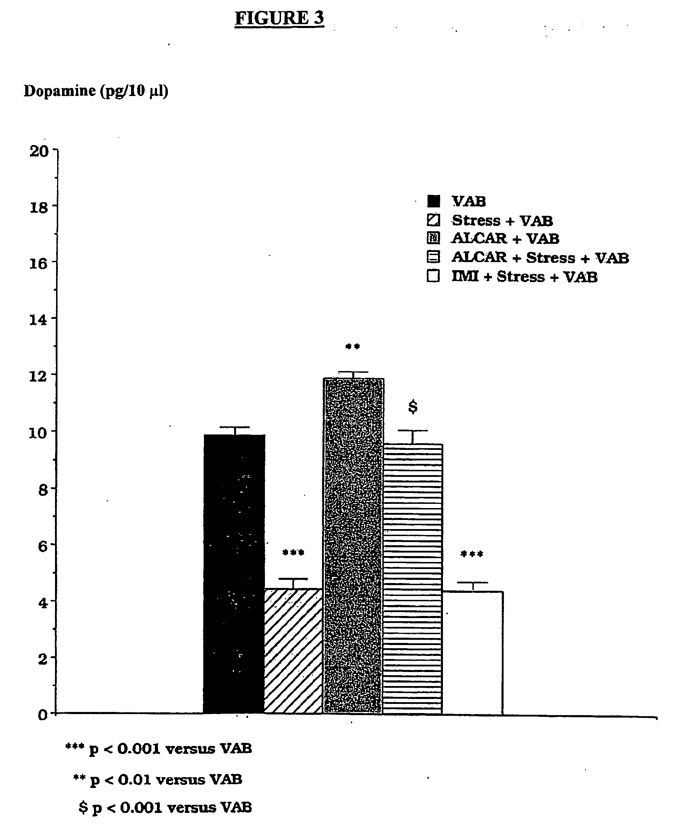

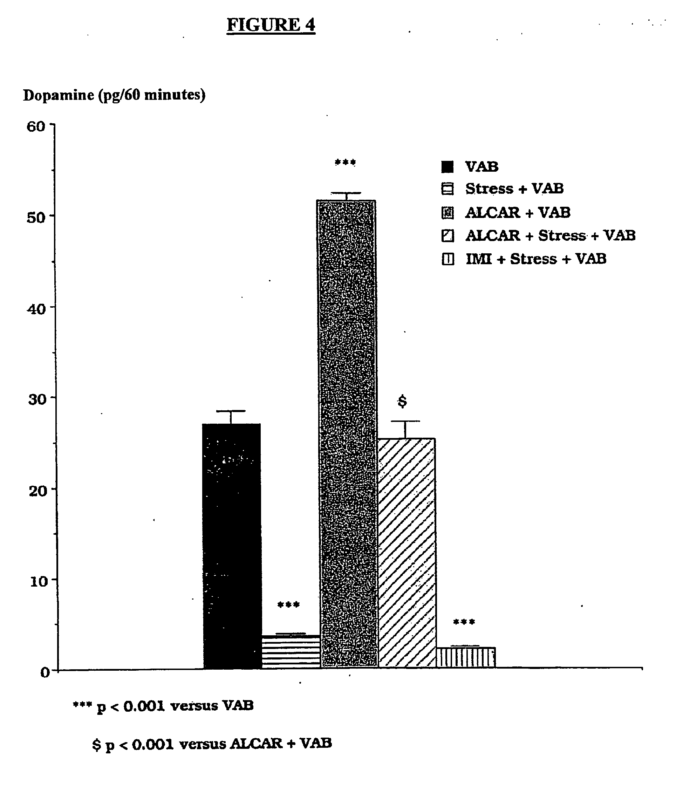

Finally, the following results are shown in Figures 3 and 4:
a) the basal levels of extraneuronal dopamine and of dopamine in the Nacs after the acute administration of cocaine were significantly lower in rats trained in the Y-maze during chronic stress exposure than in the control animals;
b) the basal levels of extraneuronal dopamine and of dopamine in the Nacs after the acute administration of cocaine were significantly higher in rats trained in the Y-maze during chronic stress exposure and treated with acetyl L-carnitine than in the control animals;
c) the basal levels of extraneuronal dopamine and of dopamine in the Nacs after the acute administration of cocaine were significantly lower in rats trained in the Y-maze during chronic stress exposure and treated with IMI than in the control rats, and superimposable to the basal levels observed in the animals exposed to chronic stress;
d) the dopamine output levels in the Nacs of those rats trained in the Y-maze during chronic stress exposure and treated with the acetyl L-carnitine were superimposable to those observed in the control group.

The treatment with IMI reversed the stress-induced escape deficit, but did not protect the rats against the negative effect of the chronic stress either on the acquisition of VAB or on the dopaminergic transmission in the Nacs.

By contrast, the rats treated with acetyl L-carnitine recovered their capacity to acquire motivated appetitive behaviour and avoid negative stimuli. Furthermore, the levels of extraneuronal dopamine in the Nacs of the rats trained for VAB during chronic stress exposure and treated with acetyl L-carnitine were superimposable to the levels recorded in the control rats, either in basal conditions or after one single administration of cocaine.

The VAB model is a model of motivated appetitive behaviour sustained by the palatable taste of vanilla sugar.

Though fed ad libitum with a standard diet, the rats ate the vanilla sugar pellets eagerly, the consumption of which induces a significant increase of dopamine output in the Nacs (Behav. Pharmacol. 8: 619-628, 1997).

When exposed to chronic stress, the rats either may or may not be attracted by vanilla sugar pellets and consume them or not.

Nevertheless, no variation occurs on the levels of extraneuronal dopamine in the limbic system of the rats eating the sweet pellets

The dopamine increase in some discrete zones of the brain such as the pre-frontal cortex and the Nacs after the exposure to an environmental stimulus has been associated to the contingent importance acquired by such stimulus when it is perceived.

The increase in monoammine output reveals what is the level of importance of a certain stimulus to the body perceiving it.

Thus, the consumption of vanilla sugar pellets in the control rats is an important stimulus, which increases the release of dopamine in the Nacs and can sustain a motivated appetitive behaviour aimed at being repeated.

By contrast, the consumption of vanilla sugar pellets is not perceived as an important stimulus by the rats exposed to stress. By consequence, the dopamine output in the Nacs does not increase and does not act as a reinforcement of motivated appetitive behaviour.

### EXAMPLE 2

In this example, the effect of a 7-day administration of acetyl L-carnitine, IMI or FLX on the serotoninergic and dopaminergic brain transmission in rats exposed to stress has been studied.

The aim of this experiment was to determine whether a 7-day exposure to stress would modify the output of dopamine and serotonine in the pre-frontal cortex (CPF) and in the shell of the nucleus accumbens (Nacs), as well as to assess the effects of acetyl L-carnitine, IMI or FLX on such modifications.

Sixty rats were divided in 6 groups of 10 animals each, as follows:
a) Control (Crt): the animals were administered physiological solution 1 ml/kg IP, BID for 8 days;
b) acetyl L-carnitine: the animals were administered acetyl L-carnitine 10 mg/kg IP, BID for 8 days;
c) Stress: the animals were exposed to the pre-test, they were tested for escape 24 hours later, and then they were exposed to the chronic stress protocol for 7 days, while receiving physiological solution (for 8 days);
d) acetyl L-carnitine + Stress: the animals were exposed to the pre-test, they were tested for escape 24 hours later, and then they were exposed to the chronic stress protocol for 7 days, while receiving acetyl L-carnitine 10 mg/kg IP, BID, for 8 days;
e) IMI + Stress: the animals were exposed to the pre-test, they were tested for escape 24 hours later, and then they were exposed to the chronic stress protocol for 7 days, while receiving IMI 5 mg/kg IP, BID for 8 days;
f) FLX + Stress: the animals were exposed to the pre-test, they were tested for escape 24 hours later, and then they were exposed to the chronic stress protocol for 7 days, while receiving FLX 5 mg/kg IP/die for 8 days.

On day 9, all the animals were implanted with dialysis probes in the Nacs; on day 10, the animals were dialysed.

The basal levels of dopamine were determined over 60 minutes.

At this point, the rats were offered 5 vanilla sugar pellets (Meal 1); 4 samples of dialysis fluid were taken over the next 60 minutes.

The rats were offered vanilla sugar one more time (Meal 2), and samples of dialysis fluid were taken over 60 minutes.

Finally, the rats were injected with cocaine 5 mg/kg IP, 0.1ml in water and samples of dialysis fluid were taken over 60 minutes.

To be able to explain the protective effect exerted by the acetyl L-carnitine on the acquisition of VAB in rats exposed to a chronic stress procedure, it has been assumed that the vanilla sugar pellets may maintain a considerable level of palatable taste in those rats exposed to chronic stress and treated with acetyl L-carnitine.

The hypothesis also assumed that the IMI would not exert such effect.

In other words, if the rats first exposed to stress and then treated with acetyl L-carnitine consumed vanilla sugar pellets and showed an increased output of dopamine in the Nacs, while the rats first exposed to stress and then treated with IMI did not, then the acetyl L-carnitine might be defined as the first compound capable to antagonize the anhedonia induced by chronic stress.

To investigate this point, an experiment was carried out to determine the effects that a 7-day administration of IMI, FLX or acetyl L-carnitine would have on the cerebral dopaminergic transmission in rats exposed to stress.

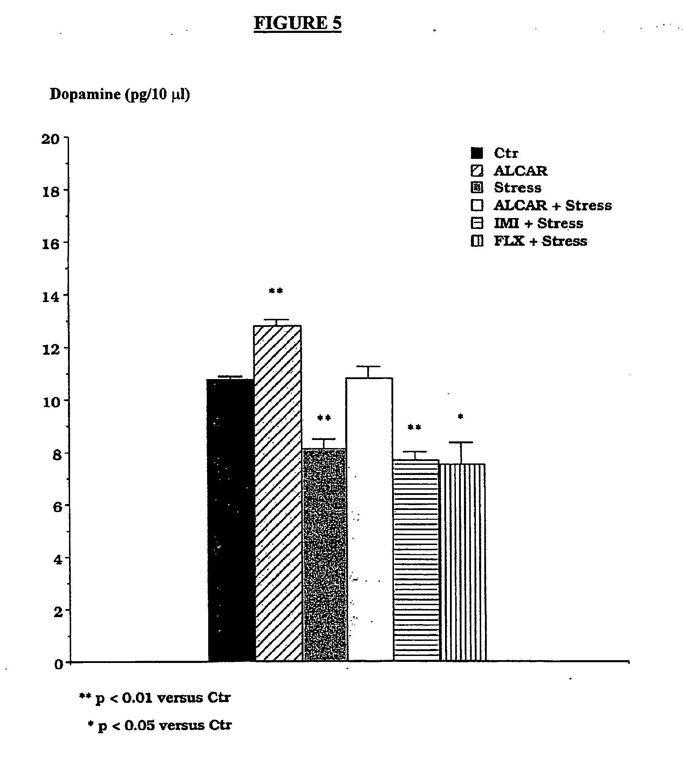

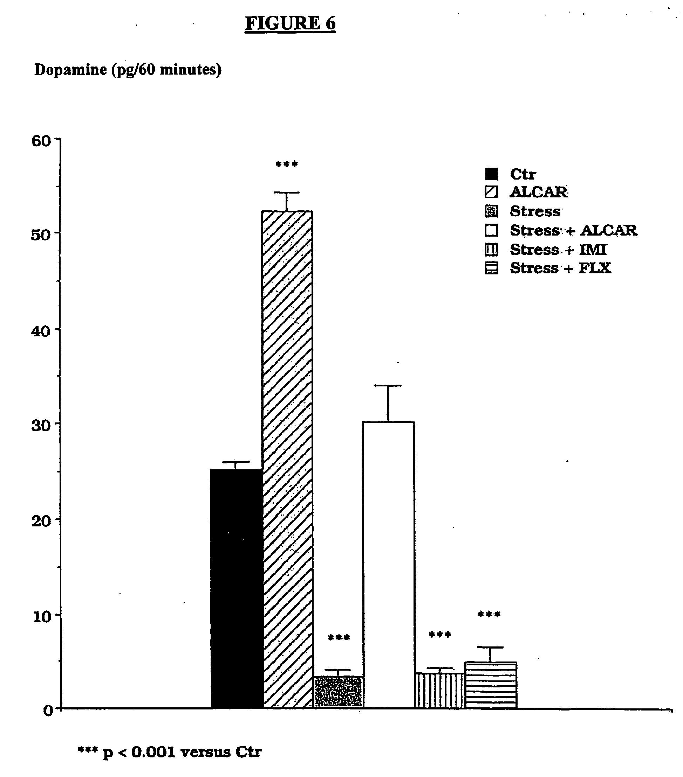

Figures 5 and 6 show that after a 7-day exposure to stress, the basal levels of dopamine in the Nacs were significantly higher in the rats treated with acetyl L-carnitine than in the control rats; furthermore, the basal levels of dopamine in the Stress group rats were significantly lower than in the controls in the same limbic zone.

Besides, the increase of dopamine in the Nacs after the acute administration of cocaine resulted to be significantly higher in the rats treated with acetyl L-carnitine and remarkably lower in the animals belonging to the Stress group than in the control rats. Neither the treatment with IMI nor the treatment with FLX were capable to protect the rats from the neurochemical effects resulting from a repeated exposure to stress.

On the contrary, the animals while exposed to stress, presented levels of extraneuronal dopamine in the NACS, superimposable to those observed in the control group both in basal conditions and after the administration of cocaine.

In agreement with the results disclosed in Neuroscience, 89: 637-641, 1999, and in Behav. Pharmacol., 8: 619-628, 1997, a small though significant increase in the release of dopamine in the Nacs was induced by the consumption of the vanilla sugar pellets in the control animals and in the acetyl L-carnitine group, and the tolerance to such effect developed quickly.

The results obtained are shown in Table 4.

**TABLE 4**

| **Group.** | **Dopamine** (max percentage increase) | |
|---|---|---|
| Control | Meal 1 | Meal 2 |
| Control | 37.3±1.1** | 8.5±4.3 |
| Stress | 5.4±3.3 | 4.6±3.9 |
| Acetyl L-carnitine | 35.7±1.8** | 6.8±5.1 |
| IMI + Stress | 3.3±2.3 | 4.1±3.5 |
| FLX + Stress | 4.6±4.2 | 3.6±4.0 |
| Acetyl L-carnitine + Stress | 286.4±16.4*** | 276.7±18.6*** |

| | | |
|---|---|---|
| Animals exposed to pre-test, escape test and then to chronic stress for 7 days during administration of ALCAR, IMI or FLX; The values represent the mean ± standard error of 10 determinations; ** = p<0.01 compared with their own basal values and with the Meal 2 values; *** = p<0.001 compared with their own basal values and with the Meal 1 values of the control group and of the acetyl L-carnitine+ Stress group. | | |

The animals belonging to the Stress group ate the two meals of vanilla sugar without showing significant modifications in the output of extraneuronal dopamine in the Nacs.

Both the rats of the IMI + stress group and of the FLX + stress group ate the vanilla sugar pellets with results similar to those of the Stress group.

The animals exposed to stress and then treated with acetyl L-carnitine eagerly consumed the two meals of vanilla sugar and showed a remarkable increase in the output of dopamine (approx 300%) after two consecutive vanilla sugar meals.

The analysis of the results obtained comparing the effects of antidepressant medications and acetyl L-carnitine in terms of VAB acquisition in stress exposure conditions leads to the following conclusions:
a) both the traditional antidepressants and the acetyl L-carnitine completely prevent both the behaviour sequelae resulting from chronic stress exposure and the related effects on the VAB acquisition, provided that the rats are also treated before being exposed to the pre-test;
b) the daily administration of IMI after the pre-test and escape test sequence, and during the training phase in the rats exposed to the chronic stress procedure completely reversed the escape deficit condition. Nevertheless, it did not protect the animals either from the negative effects of stress on the VAB acquisition or from the reduction in dopamine output in the Nacs;
c) the daily administration of acetyl L-carnitine after the pre-test and escape test sequence, and during the training phase in the rats exposed to the chronic stress procedure completely protected the animals from the negative effects of stress on the VAB acquisition. Furthermore, the levels of dopaminergic transmission in the Nacs of rats that had learnt the motivated appetitive behaviour rinforced by vanilla sugar pellets while being exposed to the stress procedure and the treatment with acetyl L-carnitine were superimposable to those observed in the control animals and significantly higher than those observed in the rats exposed to chronic stress.

The protective effect of the acetyl L-carnitine on the VAB acquisition during exposure to chronic stress is justified by the capacity to prevent the reduction of the stress-induced dopaminergic transmission in the Nacs.

Such effect was induced only by acetyl L-carnitine. In fact, neither the treatment with IMI nor the treatment with FLX for one week showed any protection on the behaviour and neurochemical sequelae (Behav. Pharmacol., 6: 562-567, 1995) (J. Neurochem, 72: 2039-2046, 1999) of exposure to stress.

On the contrary, the effect of the acetyl L-carnitine appeared reinforced by the exposure to stress, since the dopaminergic response in the Nacs to the consumption of vanilla sugar resulted remarkably higher in the group acetyl L-carnitine + Stress than in the control and acetyl L-carnitine groups.

Therefore, the acetyl L-carnitine is the only known compound capable to antagonize the decrease of the dopaminergic transmission in the Nacs resulting from a repeated exposure to stress.

The mechanism of such effect seems to be linked to its capacity of increasing the output of dopamine in the same area in the control rats.

When treated with acetyl L-carnitine, the rats exposed to chronic stress maintained their capacity to perceive a palatable food as a sufficient reinforcement to sustain motivated appetitive behaviour.

According to the results, it derives that the acetyl L-carnitine is the only known compound capable to antagonize the negative effects of the exposure to chronic stress on the acquisition of motivated appetitive behaviour.

## Claims

1. Use of an acetyl L-carnitine, or a pharmaceutically acceptable salt thereof, for the preparation of a medicament for the treatment of anhedonia.

2. The use according to claim 1, wherein the pharmaceutically acceptable salt of acetyl L-carnitine is selected in the group consisting of: chloride, bromide, orotate, acid aspartate, acid citrate, citrate magnesium, acid phosphate, fumarate and acid fumarate, fumarate magnesium, lactate, maleate and acid maleate, mucate, acid oxalate, pamoate, acid pamoate, acid sulphate, phosphate glucose, tartrate, acid tartrate, tartrate magnesium, 2-amine ethanesulphonate, magnesium 2-amine ethanesulphonate, tartrate coline and trichloroacetate.

## Patentansprüche

1. Verwendung eines Acetyl-L-Carnitins oder eines seiner pharmazeutisch verträglichen Salze zur Herstellung eines Medikaments zur Behandlung von Anhedonie.

2. Verwendung gemäß Anspruch 1, worin das pharmazeutisch verträgliche Salz von Acetyl-L-Carnitin ausgewählt ist aus der Gruppe bestehend aus: Chlorid, Bromid, Orotat, saurem Aspartat, saurem Citrat, Magnesiumcitrat, saurem Phosphat, Fumarat und saurem Fumarat, Magnesiumfumarat, Lactat, Maleat und saurem Maleat, Mucat, saurem Oxalat, Pamoat, saurem Pamoat, saurem Sulfat, Glucosephosphat, Tartrat, saurem Tartrat, Magnesiumtartrat, 2-Aminoethansulfonat, Magnesium-2-aminoethansulfonat, Cholintartrat und Trichloracetat.

## Revendications

1. Utilisation d'acétyl-L-carnitine, ou d'un sel pharmaceutiquement acceptable de celle-ci, pour la l'anhédonisme.

2. Utilisation selon la revendication 1, dans laquelle le sel pharmaceutiquement acceptable d'acétyl-L-carnitine est choisi dans le groupe consistant en : le chlorure, le bromure, l'orotate, l'aspartate acide, le citrate acide, le magnésium citrate, le phosphate acide, le fumarate et le fumarate acide, le magnésium fumarate, le lactate, le maléate et le maléate acide, le mucate, l'oxalate acide, le pamoate, le pamoate acide, le sulfate acide, le glucose phosphate, le tartrate, le tartrate acide, le magnésium tartrate, le 2-amine éthanesulfonate, le 2-amine éthanesulfonate de magnésium, la tartrate choline et le trichloroacétate.
